# EUROPEAN PATENT APPLICATION

(11) **EP 2 937 855 A1**
(43) Date of publication of application: **28.10.2015**
(21) Application number: 13865138.5
(22) Date of filing: 27.09.2013
(51) Int. Cl.: G09G 5/00, G06T 19/00, G09G 5/36

(54) **DISPLAY CONTROL APPARATUS AND STORAGE MEDIUM**

(30) Priority: 21.12.2012 JP 2012279697
(71) Applicant: Sony Corporation, Tokyo 108-0075 (JP)
(72) Inventor: SAKO, Yoichiro, Tokyo 108-0075 (JP); NAKAMURA, Takatoshi, Tokyo 108-0075 (JP); KAMADA, Yasunori, Tokyo 108-0075 (JP); KOGA, Yuki, Tokyo 108-0075 (JP); HANAYA, Hiroyuki, Tokyo 108-0075 (JP); ONUMA, Tomoya, Tokyo 108-0075 (JP); SAKODA, Kazuyuki, Tokyo 108-0075 (JP); TAKEHARA, Mitsuru, Tokyo 108-0075 (JP); KON, Takayasu, Tokyo 108-0075 (JP); HAYASHI, Kazunori, Tokyo 108-0075 (JP); ASADA, Kohei, Tokyo 108-0075 (JP); WATANABE, Kazuhiro, Tokyo 141-0022 (JP); TANGE, Akira, Tokyo 108-0075 (JP)
(74) Representative: MFG Patentanwälte Meyer-Wildhagen Meggle-Freund Gerhard PartG mbB
(86) International application number: PCT/JP2013/076408
(87) International publication number: WO 2014/097706

(57) **Abstract**

Provided is a display control apparatus including a detection unit that detects whether or not an input image contains an image of food or drink, an image creation unit that creates an image of food or drink whose exterior appearance is changed, when the detection unit detects an image of the food or drink, and a display control unit that performs control to display the image created by the image creation unit on a display unit.

## Description

### Technical Field

The present disclosure relates to a display control apparatus and a recording medium.

### Background Art

In recent years, there have been known techniques for visually informing a user by changing a display mode depending on the situation. For example, Patent Literature 1 below discloses a techniques for visually informing a driver of a situation where another car is approaching his/her car by way of a specific display format.

Further, there has also been proposed a display system in which a user's sense of satiety is manipulated visually by changing a display mode of food. For example, Non-Patent Literature 1 below proposes a system in which a sense of satiety to be obtained in eating food is manipulated by manipulating only an apparent size of food using augmented reality with no change in sizes of objects in its surroundings such that a food consumption can be changed while the sense of satiety is kept constant.

### Citation List

### Patent Literature

Patent Literature 1 JP 2012-208566A

### Non-Patent Literature

Non-Patent Literature 1 Takuji Narumi, et al. four persons, "Augmented Satiety: Controlling sense of satiety by manipulating appearance of food with augmented reality, Information Processing Society of Japan, Interaction 2012, March 15, 2012, p. 25 - 32

### Summary of Invention

### Technical Problem

In Non-Patent Literature 1 above, image processing is performed to enlarge or reduce only targeted food while a hand holding the food remains unchanged in size but transformed so as to remain consistent with the food in size. However, in order to attain the image processing for properly transforming the hand holding the food, a manner of holding the food has been required to be limited. Additionally, the image processing for properly transforming a hand holding food has had difficulty in a case where the hand continuously moves.

Therefore, a meal is required to be controlled by image processing for varying food without the use of image processing for transforming a hand holding food.

In Non-Patent Literature 1, the sense of satiety is varied by making a size sense of food to be perceived to manipulate a food intake, but a method for manipulating the food intake by a method other than varying a size of food is not referred. However, it can be considered that, in addition to the size of food, an exterior appearance including a texture, amount (number of kinds), color or the like of food may be varied to such that the food intake is controlled to be restrained or promoted.

Accordingly, the present disclosure proposes a novel and improved display control apparatus and recording medium in which a meal can be controlled by displaying an image of food or drink whose exterior appearance is changed.

### Solution to Problem

According to the present disclosure, there is provided a display control apparatus including a detection unit that detects whether or not an input image contains an image of food or drink, an image creation unit that creates an image of food or drink whose exterior appearance is changed, when the detection unit detects an image of the food or drink, and a display control unit that performs control to display the image created by the image creation unit on a display unit.

According to the present disclosure, there is provided a recording medium having a program recorded thereon, the program causing a computer to function as a detection unit that detects whether or not an input image contains an image of food or drink, an image creation unit that creates an image of food or drink whose exterior appearance is changed, when the detection unit detects an image of the food or drink, and a display control unit that performs control to display the image created by the image creation unit on a display unit.

### Advantageous Effects of Invention

As described above, according to the present disclosure, a meal can be controlled by displaying an image of food or drink whose exterior appearance is changed.

### Brief Description of Drawings

[FIG. 1] FIG. 1 is an illustration for explaining an outline of display control according to an embodiment of the present disclosure.
[FIG. 2] FIG. 2 is an illustration for explaining an exterior appearance of an HMD according to an embodiment.
[FIG. 3] FIG. 3 is a block diagram showing an exemplary internal configuration of the HMD shown in FIG. 2.
[FIG. 4] FIG. 4 is a flowchart showing display control processing according to a first embodiment.
[FIG. 5] FIG. 5 is an illustration showing an example of the image processing according to the first embodiment.
[FIG. 6] FIG. 6 is an illustration showing another example of the image processing according to the first embodiment.
[FIG. 7] FIG. 7 is a flowchart showing display control processing according to a second embodiment.
[FIG. 8] FIG. 8 is an illustration showing an example of image processing for promoting consumption prohibition according to the second embodiment.
[FIG. 9] FIG. 9 is a flowchart showing display control processing according to a third embodiment.
[FIG. 10] FIG. 10 is an illustration showing an example of image processing according to the third embodiment.
[FIG. 11] FIG. 11 is a flowchart showing display control processing according to a fourth embodiment.
[FIG. 12] FIG. 12 is an illustration for explaining a specific example of image processing according to the fourth embodiment.
[FIG. 13] FIG. 13 is a flowchart showing display control processing according to a fifth embodiment.
[FIG. 14] FIG. 14 is an illustration for explaining an outline of display control according to a sixth embodiment.
[FIG. 15] FIG. 15 is an illustration showing an example of image of food or drink contained in a menu image whose exterior appearance is changed depending on attributes of the food or drink according to the sixth embodiment.

### Description of Embodiments

Hereinafter, preferred embodiments of the present invention will be described in detail with reference to the appended drawings. Note that, in this specification and the drawings, elements that have substantially the same function and structure are denoted with the same reference signs, and repeated explanation is omitted.

A description is given in the following order.
1. Outline of display control according to an embodiment of the present disclosure
2. Configuration of HMD
3. Embodiments
3-1. First embodiment
3-2. Second embodiment
3-3. Third embodiment
3-4. Fourth embodiment
3-5. Fifth embodiment
3-6. Sixth embodiment
4. Conclusion

### «1. Outline of display control system according to an embodiment of the present disclosure»

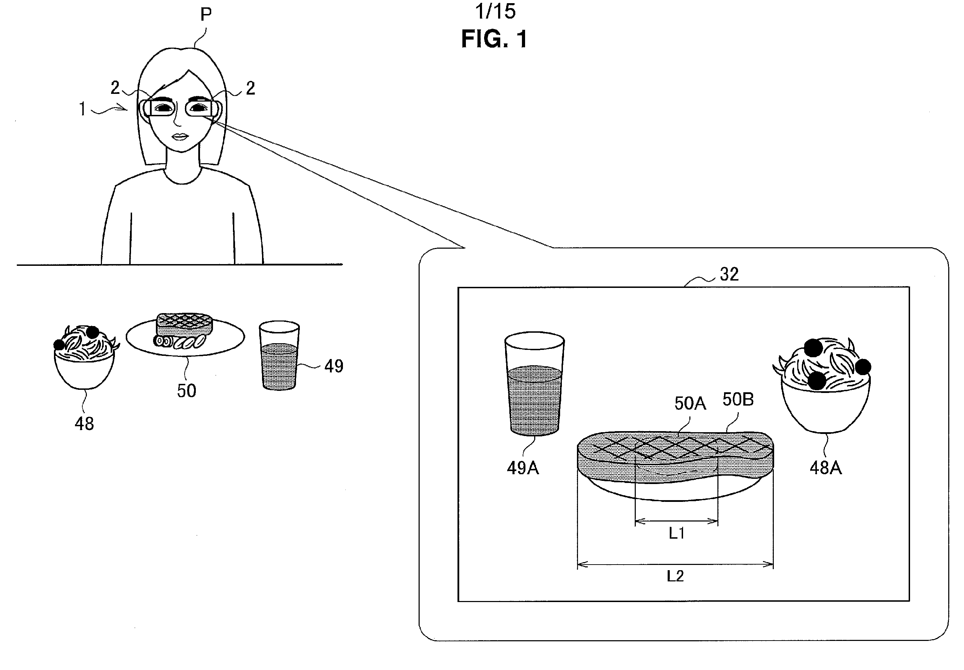

First, a description is given of an outline of display control according to an embodiment of the present disclosure with reference to FIG. 1.

FIG. 1 is an illustration for explaining an outline of display control according to an embodiment of the present disclosure. As illustrated in FIG. 1, the display control according to the embodiment is implemented by a head mounted display (HMD) 1 (display control apparatus).

The HMD 1 is a glasses-type wearable device mounted on a user P as illustrated in FIG. 1. The HMD 1 has a configuration in which a pair of display units 2 for the right and left eyes are disposed immediately in front of both eyes of the user, that is, at positions of lenses of general glasses, in the mounted state.

The display unit 2 may be a transmission type and the display unit 2 is allowed to be in a through state, that is, a transparent state or a semi-transparent state, by the HMD 1 such that there is no inconvenience in normal life even when the user P continuously wears the HMD 1 like glasses.

Here, in Non-Patent Literature 1 described above, image processing is performed to enlarge or reduce only targeted food while a hand holding the food remains unchanged in size but transformed so as to remain consistent with the food in size. However, the image processing for properly transforming a hand holding food has had difficulty in a case where the hand continuously moves.

In contrast to this, the HMD 1 (display control apparatus) according to the embodiment can control a meal by displaying an image generated using image processing for changing an exterior appearance of food or drink without the use of the image processing for transforming a hand holding food or drink.

For example, as illustrated in FIG. 1, when the user P takes a meal, the HMD 1 mounted on the user P images a beverage 49, salad 48, and steak 50 in front of his/her eyes. The HMD 1 detects an image of food or drink from a captured image and creates an image of the food or drink whose exterior appearance is changed and displays the created image on the display units 2.

At this time, the HMD 1 may detect attributes of food or drink and create an image of food or drink whose exterior appearance is changed on the basis of the detected attributes of the food or drink.

For example, the HMD 1, if an image of the beverage 49, an image of the salad 48, and an image of the steak 50 are detected from the captured image, can analyze the images to detect a beverage attribute, a vegetable attribute, and a meat attribute from the images. Then, in a case where the user P is on a diet (in abstinence), image processing is performed for enlarging a size of the image of the steak 50 from which the meat attribute is detected, for example, to give a sense of satiety owing to a visual effect more than that given by a food consumption of a real steak, preventing overeating of the meats.

Specifically, as illustrated in FIG. 1, the HMD 1 performs the image processing for changing a length L1 of the steak into a length L2 which is longer than L1 with respect to an image of the steak 50 (steak image 50A) from which the meat attribute is detected so as to create an image 32 containing a steak image 50B having enlarged size and display the created image on the display units 2. The steak image 50B may be an image entirely created to be synthesized over the image of the steak 50 (steak image 50A) or an image having a portion only different from the steak image 50A synthesized around the steak image 50A.

Even when the user P is on a diet (in abstinence), it is preferable to take in food or drink in a lot of amount from which the beverage attribute or the vegetable attribute is detected, and thus, an image from which these attributes are detected may not be subjected to the image processing for size change. Therefore, as illustrated in FIG. 1, a salad image 48A and a beverage image 49A contained in the created image 32 remain not subjected to the image processing for size change.

The outline of the display control according to the embodiment is described as above. Note the HMD 1 according to the embodiment is not limited to the size change of food or drink, but the exterior appearance including a texture, amount (number of kinds), color or the like of food or drink is changed such that food or drink intake can be also controlled to be restrained or promoted. Further, the HMD 1 according to the embodiment may change a predetermined exterior appearance of food or drink on the basis of whether or not the user is on a diet as well as on the basis of user information such as a health condition, allergy information, biological information or the like of the user.

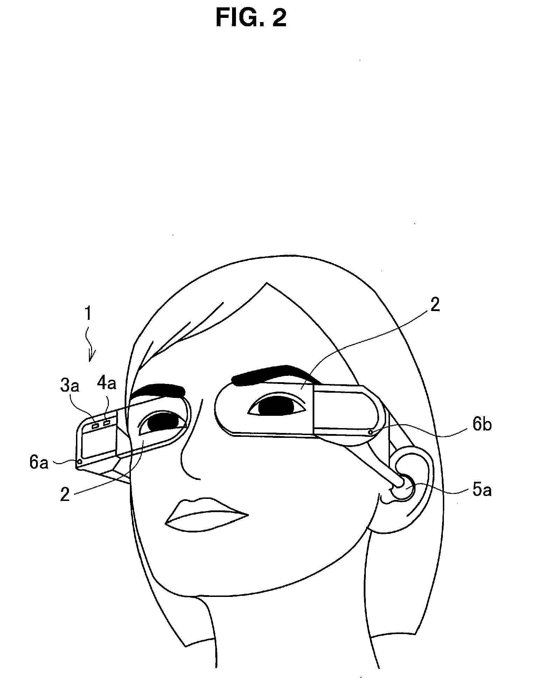

Subsequently, a description is given of a configuration of the HMD 1 (display control apparatus) implementing the display control according to the embodiment with reference to FIG. 2 to FIG. 3.

### «2. Configuration of HMD»

### <2-1. Exterior appearance of HMD>

FIG. 2 is an illustration for explaining an exterior appearance of the HMD 1 according to the embodiment. The glasses-type HMD 1 shown in FIG. 2 is also referred to as a see-through HMD, where the display units 2 may be controlled in a transmissive state.

Specifically, the HMD 1 includes a wearable unit having a structure of a frame such as surrounding half a head from right and left temporal regions to an occipital region, for example, and is put on both auditory capsules to be mounted on the use as illustrated in FIG. 2.

Further, the HMD 1 has a configuration in which a pair of display units 2 for the right and left eyes are disposed immediately in front of both eyes of the user, that is, at positions of lenses of general glasses, in the mounted state illustrated in FIG. 2. For example, liquid crystal panels are used for the display units 2. By controlling transmittance of the liquid crystal panels, the HMD 1 may be a through state, that is, a transparent state or a semi-transparent state, as illustrated in FIG. 2. By allowing the display units 2 to be in the through state, there is no inconvenience in normal life even when the user continuously wears the HMD 1 like glasses.

The display units 2 can also display a captured image of a real space imaged by the imaging lens 3a in the display units 2. The display units 2 can reproduce and display the image created by the HMD 1, contents received from external devices, contents stored in a memory medium of the HMD 1 or the like.

As illustrated in FIG. 2, the imaging lens 3a is disposed to be oriented forward in a state of being mounted on the user P such that imaging is carried out given that a direction the user looks is a subject direction. Further, a light-emitting unit 4a is disposed which illuminates an imaging direction of the imaging lens 3a. The light-emitting unit 4a is formed of a light emitting diode (LED), for example.

Although illustrated only on the left ear side in FIG. 2, a pair of earphone speakers 5a which can be inserted into the right and left ear holes of the user in the mounted state are installed.

Microphones 6a and 6b that collect external sounds are disposed on the right side of the display unit 2 for the right eye and on the left side of the display unit 2 for the left eye.

The exterior appearance of the HMD 1 shown in FIG. 1 merely illustrates an example and diverse structures can be considered for mounting the HMD 1 on the user. The HMD 1 may be formed by a mounting unit generally considered as a glasses-type or head-mounted-type. In the embodiment, at least, the display units 2 may be installed to be close in front of the eyes of the user. One pair of display units 2 may be configured to be installed to correspond to both eyes, and one display unit may also be installed to correspond to one of the eyes.

The imaging lens 3a and the light-emitting unit 4a for illumination are disposed to be oriented forward on the right eye side in the example shown in FIG. 1, but may be disposed on the left eye or both sides. The imaging lens 3a and the light-emitting unit 4a may be disposed to be oriented sideward or backward in addition to forward.

One earphone speaker 5a may be installed to be mounted only on one of the ears rather than using the right and left stereo speakers. For the microphone, one of the microphones 6a and 6b may also be used.

It can also be considered to have a configuration in which the microphones 6a and 6b or the earphone speakers 5a are not included. A configuration in which the light-emitting unit 4a is not included can also be considered.

The exterior appearance of the HMD 1 according to the embodiment is described as above. Note in the embodiment, the HMD 1 is used as an example of the display control apparatus, but the display control apparatus according to the embodiment is not limited to the HMD 1. For example, the display control apparatus according to the embodiment may be a smartphone, mobile phone terminal, personal digital assistants (PDA), personal computer (PC), tablet terminal or the like.

### <2-2. Internal configuration of HMD>

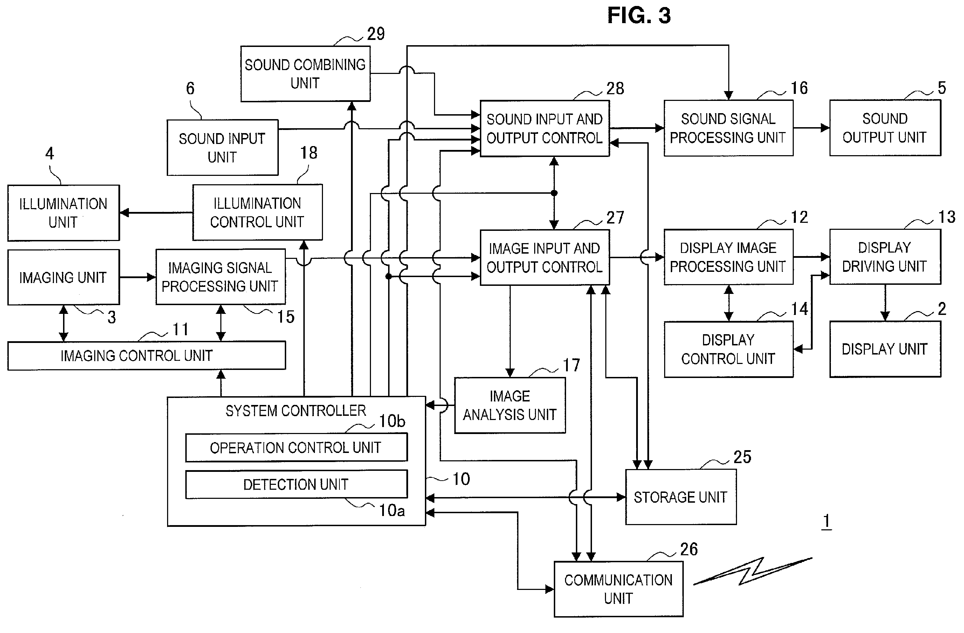

Next, the internal configuration of the HMD 1 according to the embodiment will be explained with reference to FIG. 3. FIG. 3 is a block diagram illustrating an example of the internal configuration of the HMD 1 shown in FIG. 2.

As illustrated in FIG. 3, the HMD 1 includes a display unit 2, an imaging unit 3, an illumination unit 4, a sound output unit 5, a sound input unit 6, a system controller 10, an imaging control unit 11, a display image processing unit 12, a display driving unit 13, a display control unit 14, an imaging signal processing unit 15, a sound signal processing unit 16, an image analysis unit 17, an illumination control unit 18, a storage unit 25, a communication unit 26, an image input and output control 27, a sound input and output control 28, a sound combining unit 29.

### (System controller)

The system controller 10 is configured by, for example, a microcomputer that includes a central processing unit (CPU), a read-only memory (ROM), a random access memory (RAM), a nonvolatile memory, and an interface and controls each configuration of the HMD 1.

The system controller 10, as illustrated in FIG. 2, functions as a detection unit 10a for detecting an image of food or drink from a captured image or detecting an attribute of food or drink on a basis of an image analysis result from the image analysis unit 17, and an operation control unit 10b for controlling an operation of the HMD 1.

The detection unit 10a detects an image of food or drink from a captured image on a basis of an image analysis result from the image analysis unit 17. For example, the detection unit 10a matches the image analysis result of the captured image to pattern images for detection of food or drink which are stored in the storage unit 25 in advance to be able to detect an image of food or drink. The detection unit 10a detects an attribute of food or drink on the basis of the detected image of food or drink. For example, the detection unit 10a matches the detected image of food or drink to the pattern image associated with the attribute of food or drink stored in the storage unit 25 in advance to able to detect an attribute of food or drink.

The operation control unit 10b controls operations of the HMD 1. More specifically, the operation control unit 10b according to the embodiment, in a case where the detection unit 10a detects the image of food or drink from the captured image, functions as an image creation unit which creates an image of food or drink whose exterior appearance is changed depending on the attribute of food or drink or user information. Then, the operation control unit 10b issues an instruction to the display control unit 14 to display the created image on the display units 2.

### (Imaging unit)

The imaging unit 3 includes a lens system that includes an imaging lens 3a, a diaphragm, a zoom lens, and a focus lens, a driving system that enables the lens system to execute a focus operation or a zoom operation, and a solid-state image sensor array that photoelectrically converts imaging light obtained with the lens system to generate an imaging signal. The solid-state image sensor array may be realized by, for example, a charge coupled device (CCD), a sensor array, or a complementary metal oxide semiconductor (CMOS) sensor array.

### (Imaging signal processing unit)

The imaging signal processing unit 15 includes a sample-hold and automatic gain control (AGC) circuit that performs gain adjustment or waveform shaping on a signal obtained by a solid-state image sensor of the imaging unit 3 or a video analog-to-digital (A-to-D) converter. Thus, the imaging signal processing unit 15 obtains an imaging signal as digital data. The imaging signal processing unit 15 performs a white balance process, a luminance process, a color signal process, a blur correction process, or the like on the imaging signal.

### (Imaging control unit)

The imaging control unit 11 controls the operations of the imaging unit 3 and the imaging signal processing unit 15 based on an instruction from the system controller 10. For example, the imaging control unit 11 controls ON and OFF of the operations of the imaging unit 3 and the imaging signal processing unit 15. The imaging control unit 11 is considered to perform control (motor control) on the imaging unit 3 in order to execute operations such as auto-focus, automatic exposure adjustment, diaphragm adjustment, and zoom. The imaging control unit 11 includes a timing generator and controls signal processing operations of the video A-to-D converter and the solid-state image sensor and the sample-hold and AGC circuit of the imaging signal processing unit 15 based on a timing signal generated by the timing generator. Variable control of an imaging frame rate is considered to be performed by the timing control.

The imaging control unit 11 performs control of imaging sensitivity or signal processing in the solid-state imaging element and the imaging signal processing unit 15. For example, as the control of the imaging sensitivity, gain control of a signal read from the solid-state image sensor can be performed. Alternatively, control of various coefficients of imaging signal processing at a digital data stage, black level setting control, correction amount control in a blur correction process, or the like can be performed.

### (Image input and output control)

The imaging signal (image data obtained by imaging) imaged by the imaging unit 3 and processed by the imaging signal processing unit 15 is supplied to the image input and output control 27. The image input and output control 27 controls transmission of the image data under the control of the system controller 10. In other words, the image input and output control 27 controls transmission of the image data between the imaging signal processing unit 15, display image processing unit 12, image analysis unit 17, storage unit 25, and communication unit 26. For example, the image input and output control 27 performs an operation of supplying image data (captured image) as an imaging signal processed by the imaging signal processing unit 15 to the image analysis unit 17.

### (Display image processing unit)

The display image processing unit 12 is considered as, for example, a so-called video processor and is considered to be a unit which can perform various display processes on the supplied image data. For example, luminance level adjustment, color correction, contrast adjustment, or sharpness (contour enhancement) adjustment of the image data can be performed.

### (Display driving unit)

The display driving unit 13 includes a pixel driving circuit that displays the image data supplied from the display image processing unit 12 on the display unit 2 considered as, for example, a liquid crystal display. That is, display is performed by applying a driving signal based on a video signal to each of the pixels arranged in a matrix form in the display unit 2 at predetermined horizontal and vertical driving timings. The display driving unit 13 can control a transmittance of each pixel of the display unit 2 in accordance with an instruction from the display control unit 14 to make the display unit 2 be in the through state.

### (Display control unit)

The display control unit 14 controls a processing operation of the display image processing unit 12 or an operation of the display driving unit 13 under the control of the system controller 10. Specifically, the display control unit 14 performs control such that the display image processing unit 12 performs the luminance level adjustment and the like on the image data described above.

The display control unit 14 according to the embodiment controls the operation of the display driving unit 13, in response to the control by the system controller 10, so as to display on the display units 2 the image created by the operation control unit 10b (image creation unit) of food or drink whose exterior appearance is changed.

### (Sound input unit)

The sound input unit 6 includes the microphones 6a and 6b illustrated in FIG. 2, and a microphone amplifier unit and an A-to-D converter that amplify and process sound signals obtained by the microphones 6a and 6b. The sound input unit 6 outputs sound data to the sound input and output control 28.

### (Sound input and output control)

The sound input and output control 28 controls transmission of the sound data under the control of the system controller 10. Specifically, the sound input and output control 28 controls transmission of the sound signals among the sound input unit 6, the sound signal processing unit 16, the storage unit 25, and the communication unit 26. For example, the sound input and output control 28 performs an operation of supplying the sound data obtained by the sound input unit 6 to the sound signal processing unit 16, the storage unit 25, or the communication unit 26. The sound input and output control 28 performs an operation of supplying, for example, the sound data reproduced by the storage unit 25 to the sound signal processing unit 16 or the communication unit 26. The sound input and output control 28 performs an operation of supplying, for example, the sound data received by the communication unit 26 to the sound signal processing unit 16 or the storage unit 25.

### (Sound signal processing unit)

The sound signal processing unit 16 is formed by, for example, a digital signal processor or a D-to-A converter. The sound signal processing unit 16 is supplied with the sound data obtained by the sound input unit 6 or the sound data from the storage unit 25 or the communication unit 26 via the sound input and output control 28. The sound signal processing unit 16 performs a process such as volume adjustment, sound quality adjustment, or an acoustic effect on the supplied sound data under the control of the system controller 10. The processed sound data is converted into an analog signal to be supplied to the sound output unit 5. The sound signal processing unit 16 is not limited to the configuration in which the digital signal processing is performed, but may perform signal processing using an analog amplifier or an analog filter.

### (Sound output unit)

The sound output unit 5 includes one pair of earphone speakers 5a illustrated in FIG. 2 and amplifier circuits for the earphone speakers 5a. The sound output unit 5 may be configured as a so-called bone conduction speaker. The user can further hear an external sound through the sound output unit 5, hear a sound reproduced by the storage unit 25, or hear a sound received by the communication unit 26.

### (Storage unit)

The storage unit 25 is considered to be a unit that records and reproduces data on a predetermined recording medium. The storage unit 25 is realized as, for example, a hard disk drive (HDD). Of course, the recording medium can be considered as any of various media such as a solid-state memory such as a flash memory, a memory card including a fixed memory, an optical disc, a magneto-optical disc, and a hologram memory. The storage unit 25 may be considered to have a configuration in which recording and reproduction can be performed according to an adopted recording medium.

The storage unit 25 is supplied via the image input and output control 27 with the image data (captured image) as the imaging signal which is captured by the imaging unit 3 and processed by the imaging signal processing unit 15, the image data, created by the operation control unit 10b, of food or drink whose exterior appearance is changed, and the like. The storage unit 25 is supplied via the sound input and output control 28 with the sound data obtained by the sound input unit 6 and the sound data received by the communication unit 26.

### (Communication unit)

The communication unit 26 transmits and receives data to and from the external devices. The communication unit 26 is an example of the configuration for acquiring external information. The communication unit 26 wirelessly communicates with the external devices directly or via a network access point by way of, for example, wireless Local Area Network (LAN), Wi-Fi(R) (Wireless Fidelity), infrared communication, Bluetooth(R) or the like.

### (Sound combining unit)

The sound combining unit 29 performs sound combining under the control of the system controller 10 and outputs the sound signal. The sound signal output from the sound combining unit 29 is supplied to the sound signal processing unit 16 via the sound input and output control 28 to be processed, and then is supplied to the sound output unit 5 to be output as a sound to the user.

### (Illumination unit and illumination control unit)

The illumination unit 4 includes a light-emitting unit 4a illustrated in FIG. 2 and a light-emitting circuit that allows the light-emitting unit 4a (for example, an LED) to emit light. The illumination control unit 18 allows the illumination unit 4 to perform a light emitting operation under the control of the system controller 10. The light-emitting unit 4a of the illumination unit 4 is mounted to perform illumination on the front side, as illustrated in FIG. 1, and thus the illumination unit 4 performs an illumination operation in a visual field direction of the user.

### (Image analysis unit)

The image analysis unit 17 is an example of the configuration for acquiring external information. Specifically, the image analysis unit 17 analyzes the image data and obtains information regarding an image included in the image data. The image analysis unit 17 is supplied with image data via the image input and output control 27. The image data which is a target of the image analysis in the image analysis unit 17 is the image data which is a captured image obtained by the imaging unit 3 and the imaging signal processing unit 15, the image data received by the communication unit 26, or the image data reproduced from the recording medium by the storage unit 25.

The image analysis unit 17 according to the embodiment analyzes the captured image (image data) obtained by the imaging signal processing unit 15 to perform point detection, line/contour detection, region division or the like and outputs an image analysis result to the detection unit 10a in the system controller 10.

The internal configuration of the HMD 1 according to the embodiment is described as above. Note the sound output unit 5, sound input unit 6, sound signal processing unit 16, sound input and output control 28, and sound combining unit 29 are shown as components of the sound system, but all of these are not necessarily included. The communication unit 26 is shown as a component of the HMD 1, but is not necessarily included.

According to the above configuration, the HMD 1 can display in real time on the display units 2 the image created by changing the exterior appearance of food or drink found in the captured image captured by the imaging unit 3 to control the meal of the user P. Next, a description is specifically given of which image and in which case the HMD 1 according to the embodiment creates and how to control the meal, by use of a plurality of embodiments.

### «3. Embodiments»

### <3-1. First embodiment>

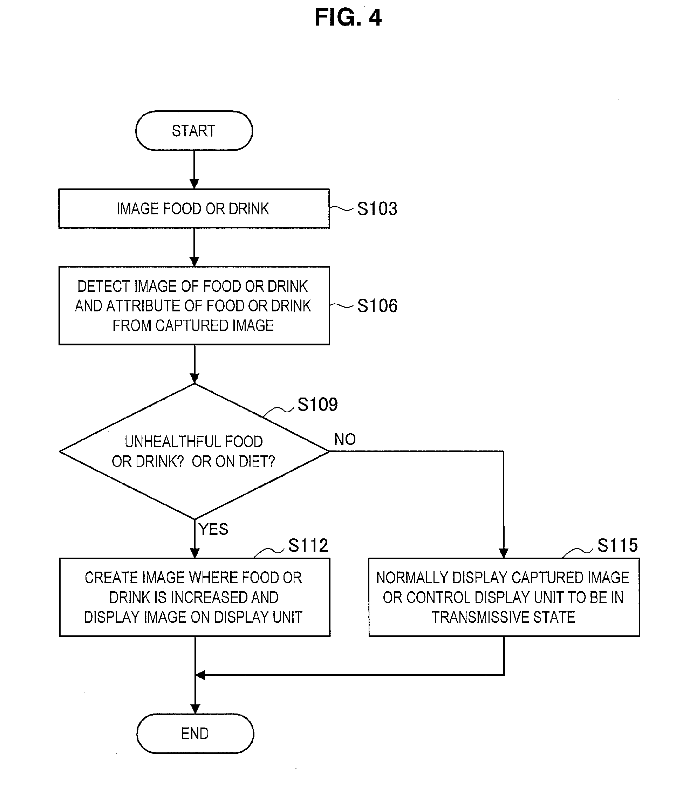

In a first embodiment, the HMD 1 creates, on the basis of the attribute of food or drink detected by the detection unit 10a, an image of the food or drink whose exterior appearance is changed. Hereinafter, a description is specifically given of display control according to the first embodiment with reference to FIG. 4 to FIG. 6.

FIG. 4 is a flowchart showing a display control processing according to the first embodiment. First, at step S103 in FIG. 4, the HMD 1 mounted on the user P images a food or drink present in an eye gaze direction of the user P by the imaging unit 3.

Subsequently, at step S106, the detection unit 10a detects an image of food or drink and an attribute of food or drink from the captured image.

Next, at step S109, the operation control unit 10b determines whether or not a case is where the attribute of food or drink detected by the detection unit 10a corresponds to an attribute of unhealthful food or drink set in advance or a case where the user P is on a diet. Whether or not the user P is on a diet may be determined based on whether or not a diet mode is set by a user operation. When the user P is on a diet, the operation control unit 10b further determines whether or not the attribute of food or drink detected by the detection unit 10a correspond to an attribute of abstinence target food or drink.

Then, in the case where the detected attribute does not correspond to the attribute of unhealthful food or drink, where the user P is not on a diet, or where the user is on a diet but the detected attributed does not correspond to the attribute of abstinence target food or drink (S109/No), the operation control unit 10b does not need to change the exterior appearance of food or drink. Accordingly, at step S115, the operation control unit 10b displays the image captured by the imaging unit 3 in real time with no change on the display units 2 or controls the display units 2 to be in the transmissive state.

On the other hand, in the case where the detected attribute corresponds to the attribute of unhealthful food or drink, or where the user P is on a diet and the detected attribute corresponds to the attribute of abstinence target food or drink (S109/Yes), the operation control unit 10b changes the exterior appearance of food or drink. Specifically, at step S112, the operation control unit 10b (image creation unit) performs on the detected image of food or drink an image processing for giving a sense of satiety more than that brought by an actual food consumption or an image processing for suppressing appetite.

The image processing for giving a sense of satiety more than that brought by an actual food consumption is, for example, an image processing that increases an amount or size of food or drink. The image processing for suppressing appetite is an image processing that changes a color or texture of food or drink into a predetermined color or texture to suppress appetite.

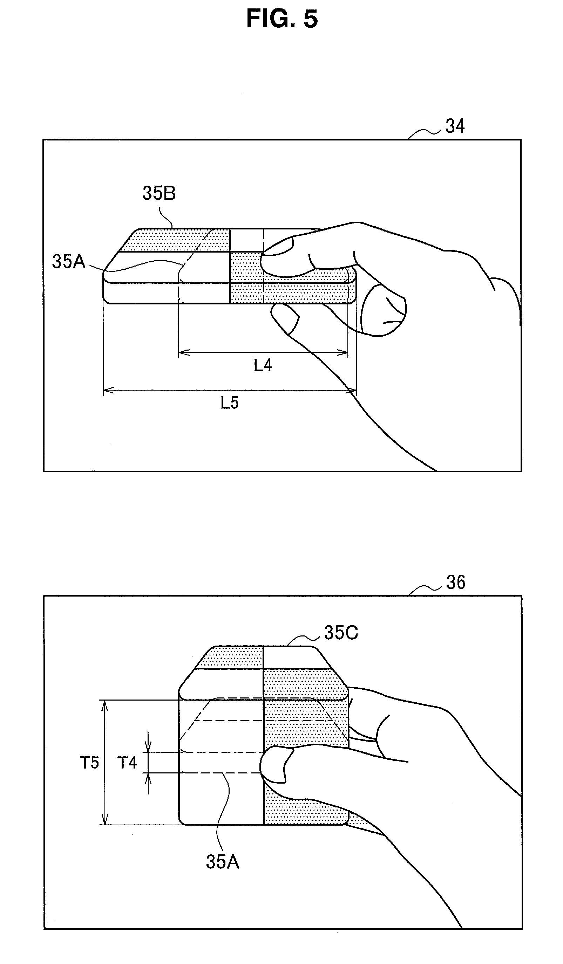

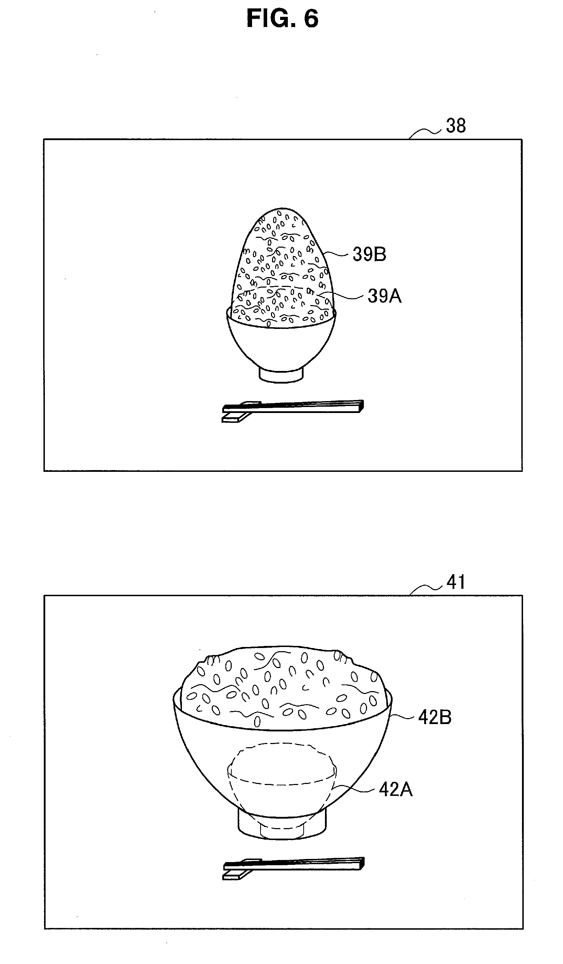

Here, a description is given of a specific example of the image processing that increases the amount or size of food or drink as the image processing for giving a sense of satiety more than that brought by an actual food consumption by use of FIG. 5 and FIG. 6.

FIG. 5 is an example of image created by an image processing for increasing a size of a piece of cookie in a case where the user holds the cookie. As in an image 34 illustrated in FIG. 5, when the user holds a piece of circular cookie in a thickness direction, the operation control unit 10b (image creation unit) creates a cookie image 35B by changing a length L4 in a radial direction of a cookie image 35A into a length L5 which is longer than L4. On the other hand, as in an image 36 illustrated in FIG. 5, when the user holds the circular cookie in the radial direction, the operation control unit 10b (image creation unit) creates a cookie image 35C by changing a thickness T4 in the thickness direction of the cookie image 35A into a thickness T5 which is thicker than T4. In this way, the size of the food or drink is changed so as not to affect held portions depending on the direction in which the user holds the food or drink, eliminating the image processing for transforming the hand.

FIG. 6 is an illustration showing an example of image created by changing an amount of rice to be increased in a case where rice is served in a bowl. For example, as in an image 38 illustrated in FIG. 6, the operation control unit 10b (image creation unit) can create a rice image 39B by changing the amount of only the rice to be increased on the basis of an rice image 39A put in the bowl. More specifically, the detection unit 10a detects an image of an object arranged around the food or drink from the captured image and the operation control unit 10b creates an image of food or drink whose exterior appearance is changed without changing an exterior appearance the object. Here, the object arranged around the food or drink includes the bowl as shown in FIG. 6 as well as a food plate such as a dish plate or chopsticks and a table.

The operation control unit 10b may create an image of the food or drink detected by the detection unit 10a as well as the food plate in which the food or drink is served, the exterior appearances of which are changed. For example, as in an image 41 illustrated in FIG. 6, the operation control unit 10b (image creation unit) may create an image 42B where an image 42A including the rice and the bowl is changed to be enlarged.

The display control according to the first embodiment is described as above. According to the first embodiment, the HMD 1 can create an image of food or drink whose exterior appearance is changed, on the basis of the attribute of the food or drink detected by the detection unit 10a to control a sense of satiety or intake amount of the user.

### <3-2. Second embodiment>

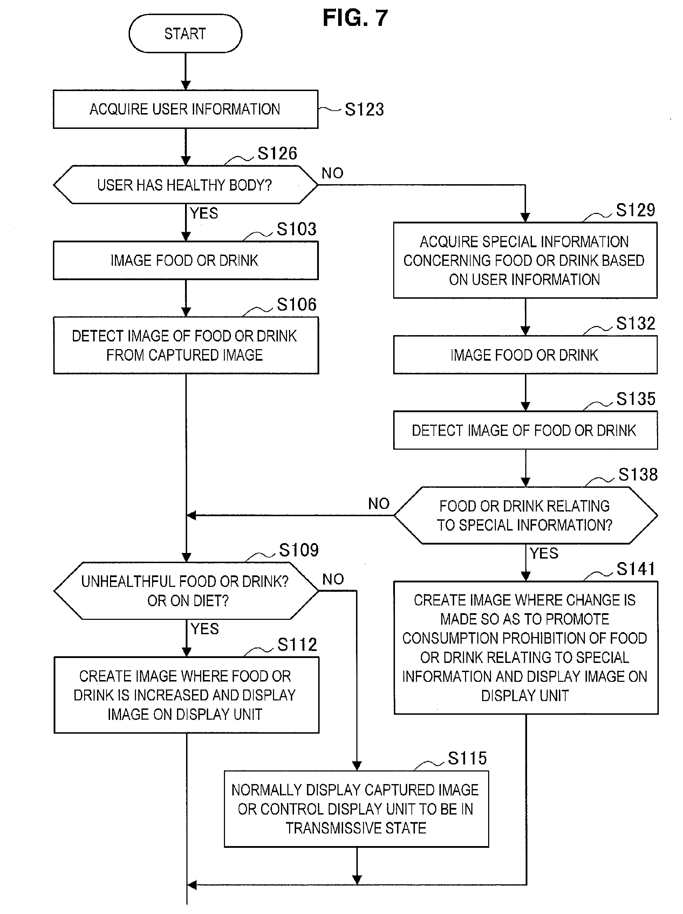

In a second embodiment, the HMD 1 creates an image of food or drink whose exterior appearance is changed, on the basis of the attribute of the food or drink detected by the detection unit 10a and the user information. Hereinafter, a description is specifically given of display control according to the second embodiment with reference to FIG. 7 to FIG. 8.

FIG. 7 is a flowchart showing display control processing according to the second embodiment. In FIG. 7, at step S123, the operation control unit 10b acquires the user information. The user information includes biological information of the user (height, weight, sweat amount, heart rate, pulse rate, blood component information), information representing the health condition (information on disease currently had), taste information on food or drink (desired taste or the like), consumption prohibition information or consumption restraint information on food or drink of the user, calorie intake information, salt intake information of the user, fat intake information of the user, sugar intake information of the user, alcohol intake information of the user, allergy information or the like. The above user information may be acquired from the user information registered in the storage unit 25 in advance, may be acquired via the communication unit 26 from the user information registered in the external devices, or may be detected and acquired in real time from various sensors mounted on the user.

Next, at step S126, the operation control unit 10b determines whether or not the user has a healthy body on the basis of the acquired user information. For example, the operation control unit 10b determines that the user has a healthy body in a case where an abnormal value is not detected from the biological information of the user to be referred, where the information representing the health condition does not contain disease information, or where the allergy information concerning food or drink is absent. The operation control unit 10b determines that the user does not have a healthy body in a case where an abnormal value is detected from the biological information of the user to be referred, where the information representing the health condition contains disease information, or where the allergy information concerning food or drink is present.

Next, if it is determined that the user does not have a healthy body (S126/No), the operation control unit 10b, at step S129, acquires special information concerning food or drink which is prohibited from being consumed or which is undesirable to be consumed on the basis of the user information. Specifically, the operation control unit 10b may extract the special information from the user information or may acquire the corresponding special information from the storage unit 25 or the external devices on the basis of the user information. For example, in a case where it is found that the user has diabetes or hyperlipidemia from the user information, the operation control unit 10b extracts as the special information the food or drink which is prohibited from being consumed or which is undesirable to be consumed in the case of diabetes or hyperlipidemia.

Next, at step S 132, the imaging unit 3 images food or drink present in the eye gaze direction of the user P.

Next, at step S135, the detection unit 10a detects an image of food or drink from the captured image. At this time, the detection unit 10a may detect together an attribute of food or drink.

Next, at step S138, the operation control unit 10b determines whether or not the image of food or drink detected by the detection unit 10a is image of food or drink relating to the special information. In other words, the operation control unit 10b determines whether or not the food or drink which is prohibited from being consumed or which is undesirable to be consumed is contained in the captured image.

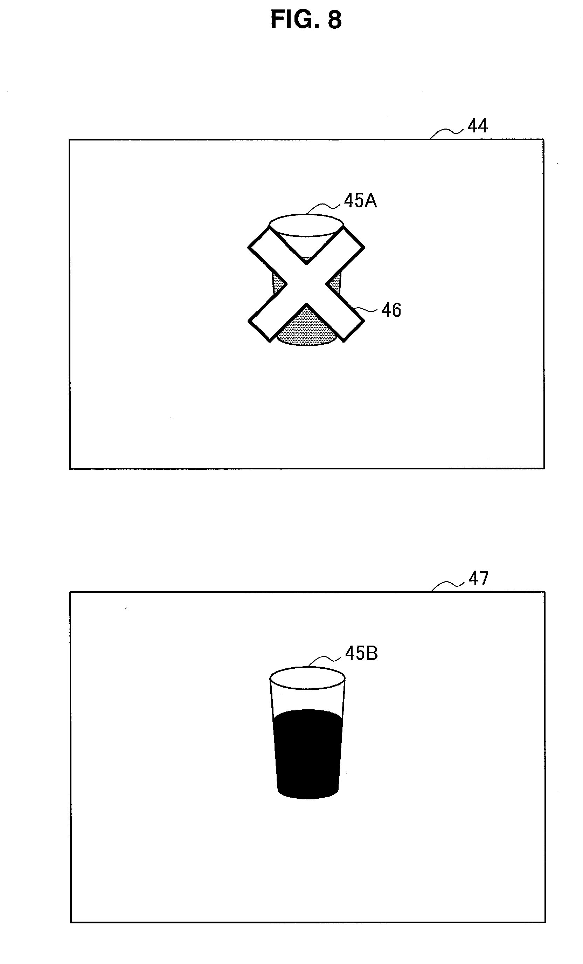

Subsequently, if the detected image is determined to be image of food or drink relating to the special information (S138/Yes), the operation control unit 10b, at step S141, creates an image of the food or drink relating to the special information, the exterior appearance of which is changed, and displays the created image on the display units 2, and promotes consumption prohibition of the food or drink. Here, FIG. 8 shows an example of image processing for promoting the consumption prohibition. As in an image 44 illustrated in FIG. 8, the operation control unit 10b may display a prohibition mark 46 to be superimposed on a captured alcoholic beverage image 45A. A in an image 47 illustrated in FIG. 8, the operation control unit 10b may create an alcoholic beverage image 45B by changing an exterior appearance of the alcoholic beverage into that having a predetermined color effective for suppressing appetite.

On the other hand, if the user is determined to have a healthy body (S126/Yes), the steps the same as those (S103 to S115) described referring to FIG. 4 are performed at steps S103 to S115.

If at step S138 the image of food or drink detected by the detection unit 10a is determined to not be the image of food or drink relating to the special information (S138/No), the operation control unit 10b performs processing shown at step S109. In other words, the operation control unit 10b determines whether or not a case is where the attribute of food or drink detected by the detection unit 10a corresponds to an attribute of unhealthful food or drink set in advance or a case is where the user P is on a diet. Then, the operation control unit 10b performs processing shown at S112 to S115 (processing the same as shown at the steps described referring to FIG. 4) depending on a determination result.

The display control according to the second embodiment is described as above. According to the second embodiment, the HMD 1 can create an image of food or drink whose exterior appearance is changed, on the basis of the user information and the attribute of the food or drink to control the meal of the user.

Note in the example described above, in the case where the food or drink relating to the special information concerning consumption of food acquired on the basis of the user information (e.g., food or drink which is prohibited from being consumed) is detected, the display control promoting the consumption prohibition of the food or drink is performed, but the display control according to the embodiment is not limited thereto.

For example, the operation control unit 10b according to the embodiment, at S112 above, may refer to the user information in changing the exterior appearance of the food or drink corresponding to the conditions shown at S109. Specifically, the operation control unit 10b can refer to the taste information of the user to perform the display control so as to increase favorite food or drink of the user such that a sense of satiety or sense of satisfaction owing to a visual effect is more effectively given, for example. The operation control unit 10b, if the user is on a diet (S109/Yes), may refer to the calorie intake information of the user to perform the image processing for suppressing appetite with respect to food or drink assumed to contain calories exceeding a calorie intake required per day.

### <3-3. Third embodiment>

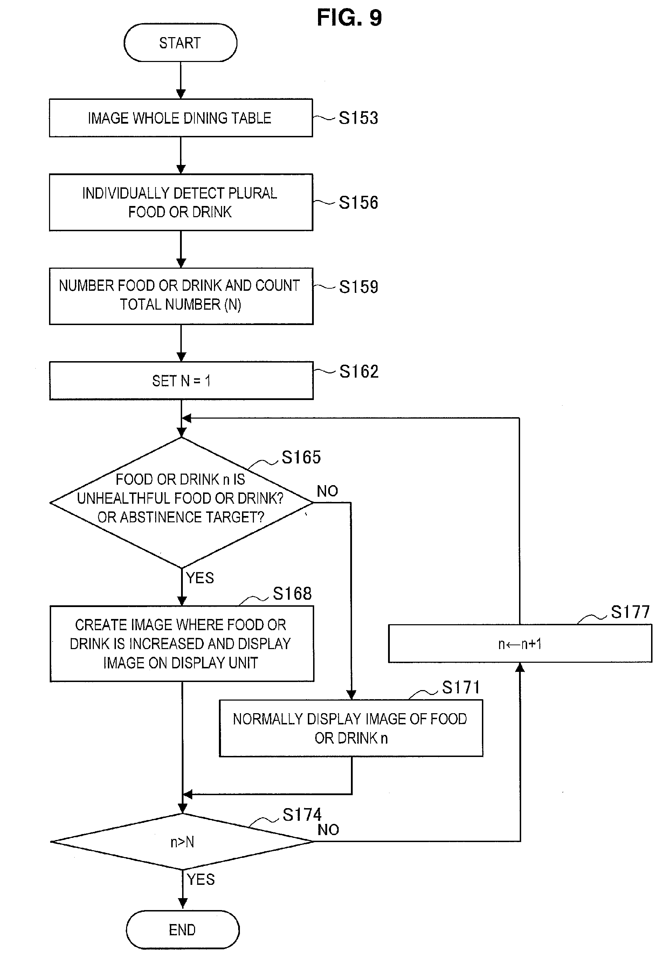

In a third embodiment, with respect to plural kinds of food or drink detected by the detection unit 10a, the HMD 1 performs the display control for changing each of exterior appearances depending on each of attributes of the food or drink. Hereinafter, a description is given of display control according to the third embodiment with reference to FIG. 9 to FIG. 10.

FIG. 9 is a flowchart showing display control processing according to the third embodiment. First, at step S153 in FIG. 9, the HMD 1 images by the imaging unit 3 a whole dining table (plate) including plural kinds of food or drink.

Next, at step S156, the detection unit 10a individually detects plural images of food or drink from the captured image. At this time, the detection unit 10a may detect together plural attributes of the food or drink.

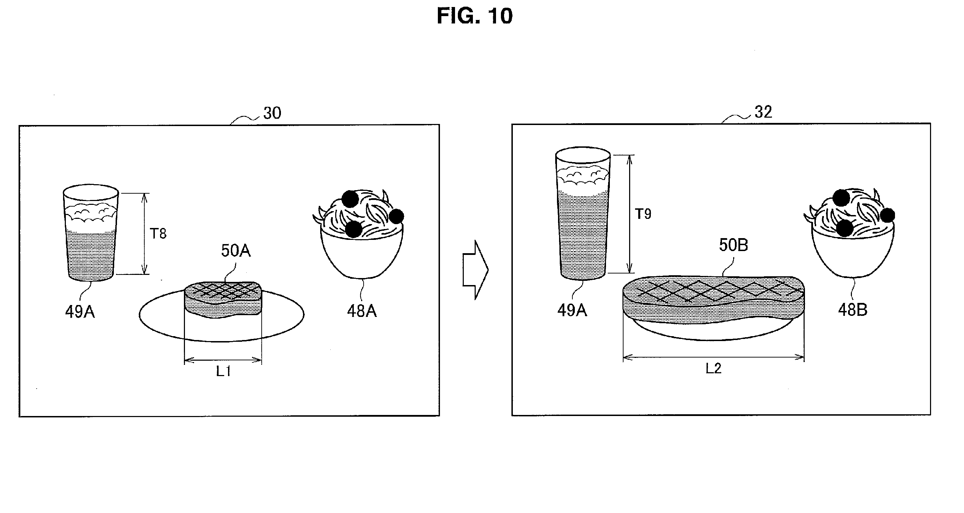

Next, at step S159, the operation control unit 10b numbers the detected plural kinds of food or drink and counts a total number N. For example, as illustrated in FIG. 10, in a case where a salad image 48A, a beverage image 49A, and a steak image 50A are detected from the captured image 30, the operation control unit 10b numbers the salad image 48A "1", the beverage image 49A "2", and the steak image 50A "3", and counts the total number "3".

Next, at step S162, the operation control unit 10b sets n = 1.

Subsequently, at step S165, the operation control unit 10b determines whether or not an attribute of a food or drink n corresponds to the attribute of unhealthful food or drink set in advance or the attribute of abstinence target food or drink. For example, if n = 1 is set, determination is made on the salad image 48A numbered "1".

Next, if none of the conditions above applies (S165/No), the operation control unit 10b, at step S171, controls an image of the food or drink n to be normally displayed on the display units 2, that is, in a state remaining as it is when captured with no change of the exterior appearance. For example, in a case where an attribute of the captured salad image 48A is determined not to apply to any of the above conditions, the salad image 48A whose exterior appearance is not changed is included in the image 32 displayed on the display units 2 as illustrated in FIG. 10.

Then at step S174, the operation control unit 10b determines whether or not "n" which is currently set is smaller than the total number "N".

Next, if "n" is smaller than "N" (S174/No), the operation control unit 10b at step S177 increments "n" currently set. This allows the operation control unit 10b to set n = 2.

Next, step S165 described above is repeated, more specifically, the operation control unit 10b makes determination on the beverage image 49A numbered "2" about whether or not an attribute of the food or drink corresponds to the attribute of unhealthful food or drink set in advance or the attribute of abstinence target food or drink.

Next, if at S165 correspondence is determined (S165/Yes), the operation control unit 10b at step S168 changes the exterior appearance of the food or drink. Specifically, the operation control unit 10b (image creation unit) performs on the detected image of food or drink the image processing for giving a sense of satiety more than that brought by an actual food consumption or the image processing for suppressing appetite.

For example, if the attribute of the beverage image 49A corresponds to the attribute of abstinence target food or drink, the operation control unit 10b creates an beverage image 49B by changing a height of a glass in which the beverage is served into a height T9 which is higher than a height T8 of the beverage image 49A in the captured image 30 as illustrated in FIG. 10. In this way, the glass is displayed to be larger than an actual size, and, in the case of the transparent glass, the glass is changed in displaying not only with the size being enlarged but also with an amount of content in the glass being increased, such that a sense of satiety more than that brought by an actual food consumption can be given to the user owing to a visual effect.

Additionally, when the glass is held, a lower half or center portion of the glass is generally held, and thus, even if an upper portion of the glass corresponding to a difference from the height T8 is a created image, the user can hold the glass without uncomfortable feeling and actually drink. As illustrated in FIG. 10, since the beverage image 49B is changed not in a width of the glass but in the height thereof to be higher, the image processing for transforming a hand of the user holding the glass or the like is not needed.

In the case where the attribute the steak image 50A corresponds to the attribute of abstinence target food or drink, the operation control unit 10b creates the steak image 50B by changing a length of the steak into a length L2 which is longer than the length L1 of the steak image 50A in the captured image 30 as illustrated in FIG. 10.

In this way, steps S165 to S177 above are repeated, when "n" currently set is equal to or larger than the total number "N" (S174/Yes), the display control for changing the exterior appearance of food or drink is ended.

The display control according to the third embodiment is described as above. According to the third embodiment, also in the case where plural images of food or drink are detected from the captured image, the HMD 1 can change an exterior appearance for each food or drink on the basis of an attribute of the food or drink to control the meal of the user.

### <3-4. Fourth embodiment>

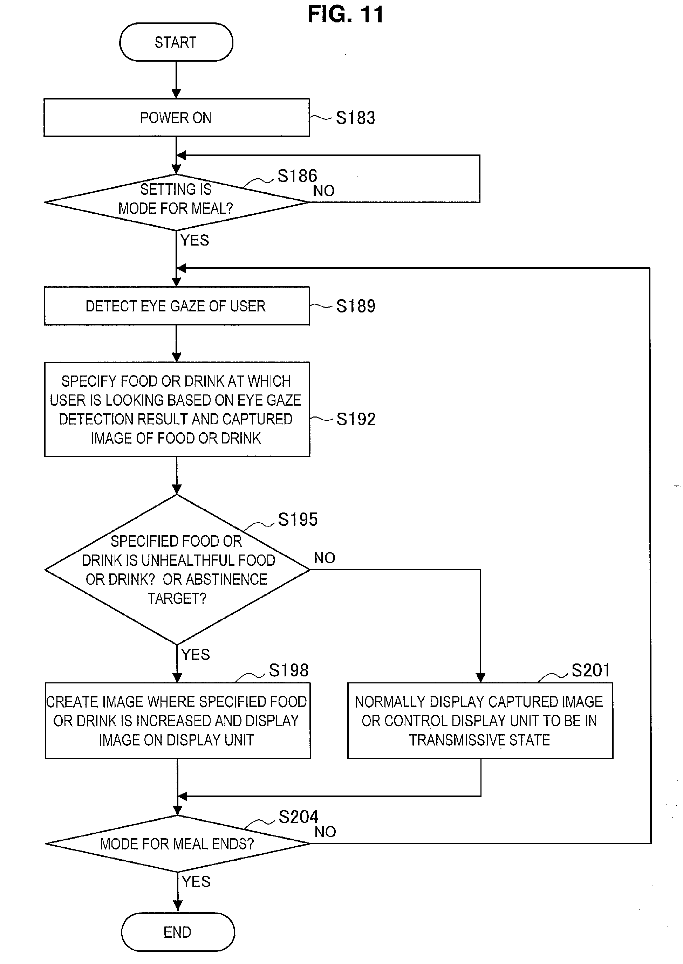

In the embodiments described above, the exterior appearance of food or drink detected from the captured image is changed depending on an attribute of food or drink or the user information, but even if the exterior appearance of food or drink is changed when the user is not looking at the food or drink, a sense of satiety or the like owing to the visual effect cannot be given. Therefore, in a fourth embodiment, in a case where the HMD 1 has an eye gaze detection function, a sense of satiety or the like owing to the visual effect is further ensured to be given to the user by performing control of changing the exterior appearance of food or drink at which the user is looking. Hereinafter, a description is given of display control according to the fourth embodiment with reference to FIG. 11 to FIG. 12.

FIG. 11 is a flowchart showing display control processing according to the fourth embodiment. First, at step S183 in FIG. 11, when the HMD 1 is powered on, the operation control unit 10b in the HMD 1, at next step S186, determines whether or not a setting is put into a mode for meal.

Next, if the setting is put into the mode for meal (S186/Yes), the HMD 1 at step S189 detects an eye gaze of the user. The eye gaze detection is detected by, for example, the operation control unit 10b on the basis of an analysis result by the image analysis unit 17 with respect to a captured image imaged by an imaging lens (not shown) which is disposed in the HMD 1 to be oriented inward such that the user' eyes are imaged when the HMD 1 is mounted. Specifically, the operation control unit 10b tracks movement of pupils of the imaged user' eyes and calculates a direction of eye gaze to be able to specify where the user is looking (eye gaze direction).

Next, at step S192, the detection unit 10a, on the basis of an eye gaze detection result by the operation control unit 10b and the captured image of food or drink imaged by the imaging unit 3, specifies the food or drink at which the user is looking. The detection unit 10a detects together an attribute of the specified food or drink.

Next, at step S195, the operation control unit 10b determines whether the attribute of the food or drink specified by the detection unit 10a corresponds to the attribute of unhealthful food or drink set in advance or corresponds to the attribute of abstinence target food or drink set in advance in a case where the user P is on a diet.

Next, if it is determined any of the conditions above applies (S195/Yes), the operation control unit 10b at step S198 creates an image of the specified food or drink whose exterior appearance is changed and displays the created image on the display units 2. Specifically, the operation control unit 10b performs the image processing for increasing an amount of the specified food or drink in order to give a sense of satiety more than that brought by an actual food consumption or the image processing for changing a color or texture of the specified food or drink in order to suppress appetite.

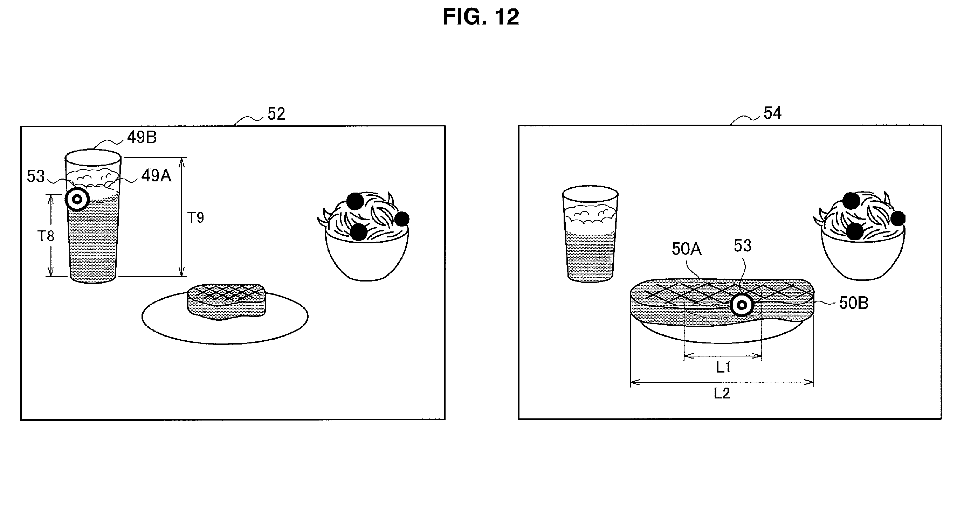

Here, a description is given of a specific example of image processing according to the embodiment by use of FIG. 12. As in an image 52 illustrated in FIG. 12, in a case where the beverage image 49A is present in an eye gaze direction 53 of the user, the operation control unit 10b creates the beverage image 49B by changing the height T8 of the glass of the beverage image 49A into the height T9 which is higher than T8 and displays the created image. As in an image 54 illustrated in FIG. 12, in a case where the steak image 50A is present in the eye gaze direction 53 of the user, the operation control unit 10b creates the steak image 50B by changing the length L1 of the steak of the steak image 50A into the length L2 which is longer than L1 and displays the created image. Note the displays of the eye gaze direction 53 illustrated in the images 52 and 54 in FIG. 12 are shown for convenience of description, and not necessarily displayed on the display units 2. The operation control unit 10b according to the embodiment can perform control to display or hide the eye gaze direction 53 depending on the setting.

The display control according to the fourth embodiment is described as above. According to the fourth embodiment, the HMD 1 can create an image of food or drink present in the eye gaze direction of the user, that is, food or drink at which the user is looking to eat and drink, the exterior appearance of which is changed, on the basis of an attribute of the food or drink to control a sense of satiety or intake amount of the user.

### <3-5. Fifth embodiment>

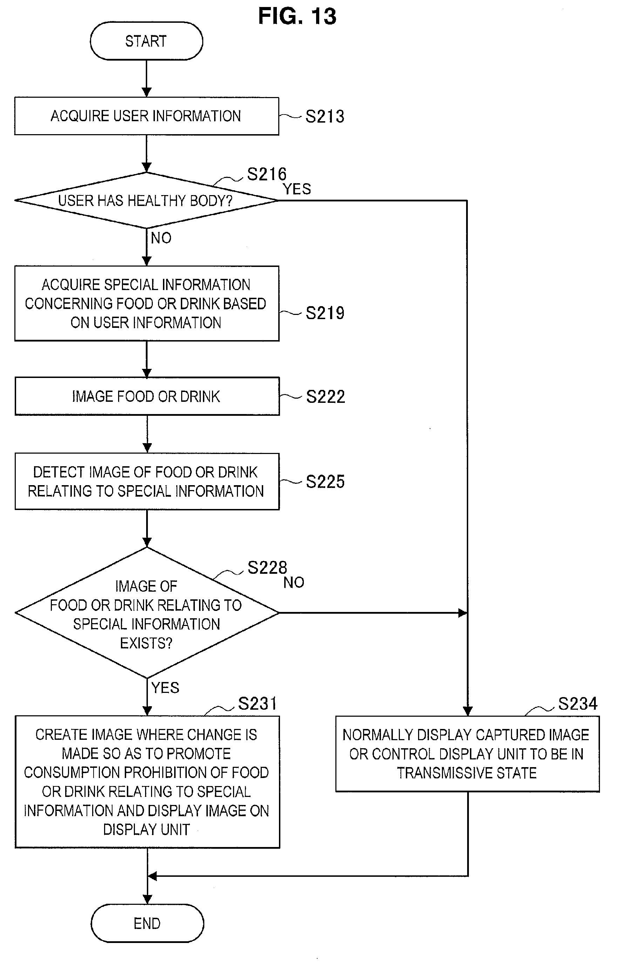

The second embodiment described above explains that the processing for changing the exterior appearance is performed on food or drink other than food or drink relating to the special information (S109 to S112 shown in FIG. 7), and at that time, the user information (taste information or the like) may be referred to, but the display control according to the present disclosure is not limited thereto. For example, in a the fifth embodiment described next, the HMD 1 may perform the display control so as to change an exterior appearance of food or drink only relating to the special information acquired from the user information. Hereinafter, a description is specifically given of display control according to the fifth embodiment with reference to FIG. 13.

FIG. 13 is a flowchart showing display control processing according to the fifth embodiment. At step S213 in FIG. 13, the operation control unit 10b acquires the user information.

Next, at step S216, the operation control unit 10b determines whether or not the user has a healthy body on the basis of the acquired user information.

Next, if the user is determined not to have a healthy body (S216/No), the operation control unit 10b, at step S219, acquires the special information concerning food or drink which is prohibited from being consumed or which is undesirable to be consumed on the basis of the user information.

Next, at step S222, the imaging unit 3 images food or drink present in the eye gaze direction of the user P.

Next, at step S225, the detection unit 10a detects an image of food or drink relating to the special information from the captured image. In other words, the operation control unit 10b detects an image of food or drink which is prohibited from being consumed or which is undesirable to be consumed from the captured image.

Subsequently, if an image of food or drink relating to the special information is detected (S228/Yes), the operation control unit 10b, at step S231, creates an image of the food or drink relating to the special information, the exterior appearance of which is changed, and displays the created image on the display unit 2, to promote the consumption prohibition of the food or drink.

On the other hand, if the user is determined to have a healthy body (S216/Yes) and if an image of food or drink relating to the special information is not detected (S228/No), the operation control unit 10b displays the image captured by the imaging unit 3 in real time with no change on the display units 2 or controls the display units 2 to be in the transmissive state.

As described above, according to the fifth embodiment, the display control can be performed so as to change the exterior appearance of the food or drink only relating to the special information acquired from the user information.

### <3-6. Sixth embodiment>

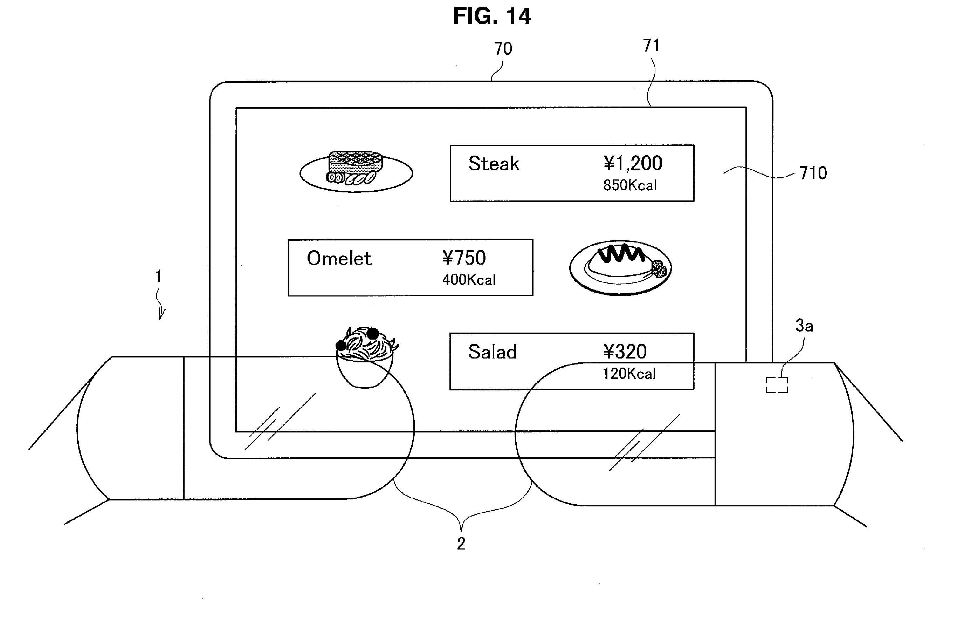

In the embodiments described above, the HMD 1 performs the display control in which food or drink in front of the user which he/she is going to eat is imaged by the imaging unit 3 and an exterior appearance of the food or drink found in the captured image is changed. However, according to the present disclosure, the display control is not limited thereto, and even in a case where the imaging unit 3 images (a photograph of) food or drink presented in a form of a paper medium or an electronic medium, the HMD 1 can also perform control such that an exterior appearance of the food or drink is changed and displayed on the display units 2. Hereinafter, a description is specifically given of display control according to a sixth embodiment for performing change processing on an exterior appearance also with respect to a photograph of food or drink in this way with reference to FIG. 14 to FIG. 15.

FIG. 14 is an illustration for explaining an outline of display control according to the sixth embodiment. As illustrated in FIG. 14, the HMD 1 according to the embodiment images a menu image 710 of food or drink at which the user is looking by the imaging lens 3a which is disposed to be oriented outward for imaging in the eye gaze direction of the user. The menu image 710, as illustrated in FIG. 14, is displayed on a display unit 71 of a tablet-type electronic terminal 70. The menu image 710 displays, as an example of menu, photographs of a steak, omelet, and salad.

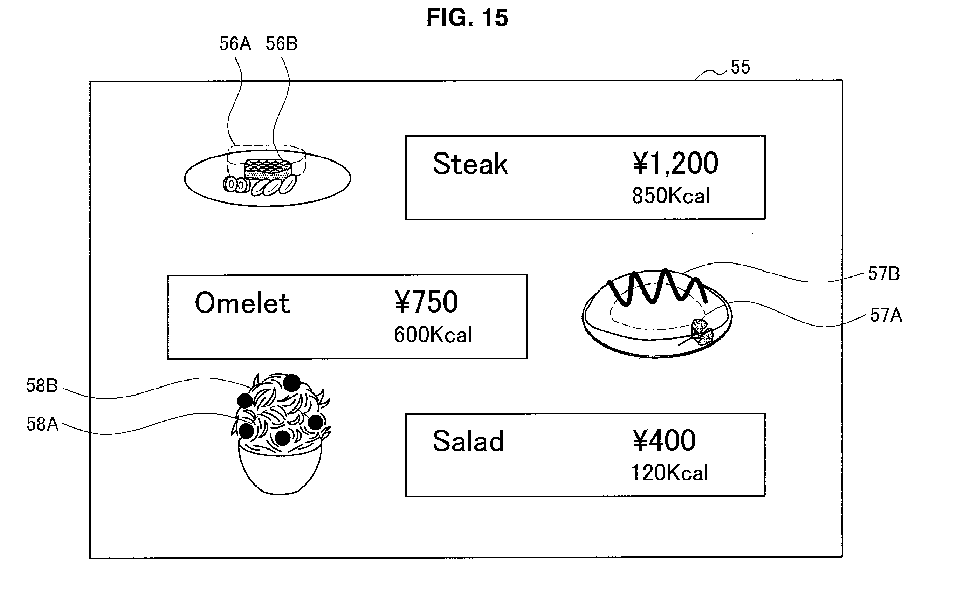

The HMD 1 according to the embodiment displays on the display units 2 an image of food or drink contained in the menu image 710 imaged by the imaging lens 3a, the exterior appearance of which is changed depending on attributes of the food or drink. Here, FIG. 15 shows an example of the image of food or drink contained in the menu image 710, the exterior appearance of which is changed depending on attributes of the food or drink.

An image 55 illustrated in FIG. 15 is an image created by the operation control unit 10b in the HMD 1 that changes the exterior appearance of food or drink depending on the attribute of food or drink detected by the detection unit 10a from the captured image which is obtained by imaging the menu image 710. The HMD 1 displays the image 55 created in this way on the display units 2.

More specifically, for example, in a case where the steak is an abstinence target food or drink, the operation control unit 10b creates a steak image 56B where a steak image 56A is made smaller so that the steak is refrained from being ordered (for suppressing appetite for the steak) as illustrated in FIG. 15.

In a case where the omelet does not correspond to any of unhealthful food or drink and abstinence target food or drink which are registered in advance, the operation control unit 10b creates an omelet image 57B where an omelet image 57A is made larger so as to promote an order of the omelet (to project a sense of value). Further, in a case where the salad does not correspond to any of unhealthful food or drink abstinence target food or drink which are registered in advance, the operation control unit 10b creates a salad image 58B where a salad image 58A is made larger so as to promote an order of the salad (to project a sense of value).

This makes it possible that, in ordering a meal in a restaurant or the like, for example, the HMD 1 changes an exterior appearance of food or drink listed in a menu so that the meal is refrained from being ordered if the user is on a diet or the meal to be ordered is unhealthful food or drink. The HMD 1 can change an exterior appearance of food or drink listed in a menu so as to promote an order, as for healthful food or drink.

### <4. Conclusion>

As described above, the display control according to the embodiment can change in real time an exterior appearance of food or drink the user is going to eat to control the meal. For example, the HMD 1 according to the embodiment can increase the size or amount of food or drink to give a sense of satiety more than that brought by an actual food consumption owing to the visual effect. This can suppress an intake amount of food or drink with no stress in the case where the user is on a diet, or a meal to be consumed is unhealthful food or drink. The HMD 1 can change a color or texture of food or drink into a predetermined color or texture for suppressing appetite to restrain an intake of predetermined food or drink with no stress.

The preferred embodiments of the present disclosure have been described above with reference to the appended drawings, but the present technology is not limited to the above examples. It is apparent to those skilled in the art that various modifications and corrections can be made within the scope of the technical spirit and essence described in the scope of the claims, and the modifications and the corrections are, of course, construed to pertain to the technical scope of the present disclosure.

For example, a computer program can also be produced for making hardware built in the HMD 1 such as a CPU, ROM, RAM and the like to exert the above described functions of the HMD 1. A computer-readable memory medium is also provided which has the computer program stored therein.

In a case where the HMD 1 according to the embodiment has a function to generate a predetermined flavor, the meal can be controlled also by the flavor, in addition to by the change of the exterior appearance of food or drink described above. For example, in the case where the user is on a diet, or the user is going to eat unhealthful food or drink, the HMD 1 generates, as a flavor for suppressing appetite, a grapefruit flavor, patchouli flavor, or cedarwood flavor.

The HMD 1 according to the embodiment performs the display control so as to, in response to decrease in an amount of the actual food or drink as the user eats and drinks, decrease an amount of the food or drink to be displayed on display units 2 in the created image having been created by changing the exterior appearance of food or drink.

For example, if an image is created by changing an amount of food or drink to be increased, the operation control unit 10b in the HMD 1 may perform the display control such that the amount decreases more in the created image displayed on the display units 2 than in the actual food or drink. If an image is created by changing an amount of food or drink to be increased, the operation control unit 10b in the HMD 1 may make a change such that when the actual food or drink decreases to below a predetermined amount, the amount of food or drink in the created image displayed on the display units 2 becomes equal to the amount of the actual food or drink.

In this way, even in the case of changing an amount of food or drink to be increased, when the actual food or drink decreases, the amount of food or drink to be displayed on the display units 2 also decreases, the exterior appearance of the food or drink having been changed, the user can take a meal without uncomfortable feeling.

The embodiments above use the HMD 1 as an example of the display control apparatus, but the display control apparatus according to the embodiment is not limited to the HMD 1, and may be a smartphone and a display control system formed of a glasses-type display, for example. The smartphone (display control apparatus) can be connected with the glasses-type display via a wireless/wired communication to transmit and receive the data.

Here, the glasses-type display, similarly to the HMD 1 shown in FIG. 2, includes a wearable unit having a structure of a frame such as surrounding half a head from right and left temporal regions to an occipital region, and is put on both auditory capsules to be mounted on the use. The display has a configuration in which a pair of display units for the right and left eyes are disposed immediately in front of both eyes of the user, that is, at positions of lenses of general glasses, in the mounted state. By controlling transmittance of the liquid crystal panels of the display units 2, the HMD 1 may be a through state, that is, a transparent state or a semi-transparent state such that there is no inconvenience in normal life even when the user continuously wears the HMD 1 like glasses.

The glasses-type display, similar to the HMD 1 shown in FIG. 2, has an imaging lens disposed thereto for imaging in the eye gaze direction of the user in the mounted state, and the glasses-type display transmits the captured image to the smartphone (display control apparatus).

The smartphone (display control apparatus), which has functions the same as the detection unit 10a and operation control unit 10b described above, detects an image and attribute of food or drink from the captured image to create an image of food or drink whose exterior appearance is changed depending on the attribute of food or drink or the user information.

Then, the smartphone (display control apparatus) transmits the created image to the glasses-type display, and the image of food or drink whose exterior appearance is changed is displayed on a display unit on the glasses-type display.

Application to a glasses-type device may be considered, the glasses-type device having a form similar to the glasses-type display but not having the display function. In this case, food or drink is imaged by a camera disposed to the glasses-type device for imaging in the eye gaze direction of a wearer (user) and the captured image is transmitted to the smartphone (display control apparatus). Then, the smartphone (display control apparatus) creates an image of food or drink whose exterior appearance is changed depending on the attribute of food or drink found in the captured image or the user information and displays the created image on the display unit of the smartphone.

Additionally, the present technology may also be configured as below.
(1)
   A display control apparatus including:
   a detection unit that detects whether or not an input image contains an image of food or drink;
   an image creation unit that creates an image of food or drink whose exterior appearance is changed, when the detection unit detects an image of the food or drink; and
   a display control unit that performs control to display the image created by the image creation unit on a display unit.
(2)
   The display control apparatus according to (1), wherein
   the detection unit further detects an attribute of the food or drink, and
   the image creation unit creates an image of the food or drink whose exterior appearance is changed on the basis of the attribute of the food or drink detected by the detection unit.
(3)
   The display control apparatus according to (1), wherein
   the detection unit detects attributes of plural kinds of food or drink detected from the input image, and
   the image creation unit selectively creates images of the plural kinds of food or drink whose exterior appearances are each changed on the basis of the attributes of the plural kinds of food or drink.
(4)
   The display control apparatus according to (2) or (3), wherein
   in a case where the attribute of the food or drink corresponds to an attribute of unhealthful food or drink set in advance or corresponds to an attribute of abstinence target food or drink, the image creation unit creates an image where an amount of the food or drink is increased, a size of the food or drink is increased, or a color or texture of the food or drink is changed into a predetermined color or texture for suppressing appetite.
(5)
   The display control apparatus according to any one of (2) to (4), wherein
   the image creation unit further creates an image of the food or drink whose exterior appearance is changed on the basis of user information.
(6)
   The display control apparatus according to (5), wherein
   the user information is at least any of biological information of a user, information representing a health condition of a user, taste information on food or drink of a user, consumption prohibition information or consumption restraint information on food or drink of a user, calorie intake information of a user, salt intake information of a user, fat intake information of a user, sugar intake information of a user, alcohol intake information of a user, and allergy information of a user.
(7)
   The display control apparatus according to any one of (1) to (5), wherein
   the image creation unit creates an image of the food or drink whose exterior appearance is changed on the basis of user information.
(8)
   The display control apparatus according to (7), wherein
   the user information is at least any of biological information of a user, information representing a health condition of a user, taste information on food or drink of a user, consumption prohibition information or consumption restraint information on food or drink of a user, calorie intake information of a user, salt intake information of a user, fat intake information of a user, sugar intake information of a user, alcohol intake information of a user, and allergy information of a user.
(9)
   The display control apparatus according to any one of (1) to (5), wherein
   the detection unit detects an image of food or drink which a user is prohibited or restrained from consuming from the input image on the basis of user information, and
   the image creation unit creates an image where the food or drink which is prohibited or restrained from being consumed is changed so as to promote prohibition or restraint of consumption.
(10)
   The display control apparatus according to (9), wherein
   the user information is at least any of biological information of a user, information representing a health condition of a user, taste information on food or drink of a user, consumption prohibition information or consumption restraint information on food or drink of a user, calorie intake information of a user, salt intake information of a user, fat intake information of a user, sugar intake information of a user, alcohol intake information of a user, and allergy information of a user.
(11)
   The display control apparatus according to any one of (1) to (10), wherein
   the input image is a captured image imaged by an imaging unit in real time.
(12)
   The display control apparatus according to (11), wherein
   the captured image is a captured image obtained by imaging food or drink present in a real space.
(13)
   The display control apparatus according to (11), wherein
   the captured image is a captured image obtained by imaging a paper medium or an electronic medium in which food or drink is presented.
(14)
   The display control apparatus according to any one of (1) to (13), wherein
   the detection unit detects an image of an object arranged around the food or drink from the input image, and
   the image creation unit creates an image of the food or drink whose exterior appearance is changed while an exterior appearance of the object is not changed.
(15)
   The display control apparatus according to any one of (1) to (14), wherein
   the detection unit detects an image of a food plate in which the food or drink is served from the input image, and
   the image creation unit creates an image of the food plate and the food or drink whose exterior appearances are changed.
(16)
   The display control apparatus according to any one of (1) to (15), wherein
   the detection unit detects food or drink present in an eye gaze direction of a user, and
   the image creation unit creates an image of the food or drink present in the eye gaze direction of the user detected by the detection unit, an exterior appearance of which is changed.
(17)
   A recording medium having a program recorded thereon, the program causing a computer to function as:
   a detection unit that detects whether or not an input image contains an image of food or drink;
   an image creation unit that creates an image of food or drink whose exterior appearance is changed, when the detection unit detects an image of the food or drink; and
   a display control unit that performs control to display the image created by the image creation unit on a display unit.

### Reference Signs List

- 1: Head mounted display (HMD)
- 2: display unit
- 3: imaging unit
- 3a: imaging lens
- 4: illumination unit
- 4a: light-emitting unit
- 5: sound output unit
- 6: sound input unit
- 10: system controller
- 10a: detection unit
- 10b: operation control unit
- 11: imaging control unit
- 12: display image processing unit
- 13: display driving unit
- 14: display control unit
- 15: imaging signal processing unit
- 16: sound signal processing unit
- 17: image analysis unit
- 18: illumination control unit
- 25: storage unit
- 26: communication unit
- 27: image input and output control
- 28: sound input and output control
- 29: sound combining unit

## Claims

1. A display control apparatus comprising:
a detection unit that detects whether or not an input image contains an image of food or drink;
an image creation unit that creates an image of food or drink whose exterior appearance is changed, when the detection unit detects an image of the food or drink; and
a display control unit that performs control to display the image created by the image creation unit on a display unit.

2. The display control apparatus according to claim 1, wherein
the detection unit further detects an attribute of the food or drink, and
the image creation unit creates an image of the food or drink whose exterior appearance is changed on the basis of the attribute of the food or drink detected by the detection unit.

3. The display control apparatus according to claim 1, wherein
the detection unit detects attributes of plural kinds of food or drink detected from the input image, and
the image creation unit selectively creates images of the plural kinds of food or drink whose exterior appearances are each changed on the basis of the attributes of the plural kinds of food or drink.

4. The display control apparatus according to claim 2, wherein
in a case where the attribute of the food or drink corresponds to an attribute of unhealthful food or drink set in advance or corresponds to an attribute of abstinence target food or drink, the image creation unit creates an image where an amount of the food or drink is increased, a size of the food or drink is increased, or a color or texture of the food or drink is changed into a predetermined color or texture for suppressing appetite.

5. The display control apparatus according to claim 2, wherein
the image creation unit further creates an image of the food or drink whose exterior appearance is changed on the basis of user information.

6. The display control apparatus according to claim 5, wherein
the user information is at least any of biological information of a user, information representing a health condition of a user, taste information on food or drink of a user, consumption prohibition information or consumption restraint information on food or drink of a user, calorie intake information of a user, salt intake information of a user, fat intake information of a user, sugar intake information of a user, alcohol intake information of a user, and allergy information of a user.

7. The display control apparatus according to claim 1, wherein
the image creation unit creates an image of the food or drink whose exterior appearance is changed on the basis of user information.

8. The display control apparatus according to claim 7, wherein
the user information is at least any of biological information of a user, information representing a health condition of a user, taste information on food or drink of a user, consumption prohibition information or consumption restraint information on food or drink of a user, calorie intake information of a user, salt intake information of a user, fat intake information of a user, sugar intake information of a user, alcohol intake information of a user, and allergy information of a user.

9. The display control apparatus according to claim 1, wherein
the detection unit detects an image of food or drink which a user is prohibited or restrained from consuming from the input image on the basis of user information, and
the image creation unit creates an image where the food or drink which is prohibited or restrained from being consumed is changed so as to promote prohibition or restraint of consumption.

10. The display control apparatus according to claim 9, wherein
the user information is at least any of biological information of a user, information representing a health condition of a user, taste information on food or drink of a user, consumption prohibition information or consumption restraint information on food or drink of a user, calorie intake information of a user, salt intake information of a user, fat intake information of a user, sugar intake information of a user, alcohol intake information of a user, and allergy information of a user.

11. The display control apparatus according to claim 1, wherein
the input image is a captured image imaged by an imaging unit in real time.

12. The display control apparatus according to claim 11, wherein
the captured image is a captured image obtained by imaging food or drink present in a real space.

13. The display control apparatus according to claim 11, wherein
the captured image is a captured image obtained by imaging a paper medium or an electronic medium in which food or drink is presented.

14. The display control apparatus according to claim 1, wherein
the detection unit detects an image of an object arranged around the food or drink from the input image, and
the image creation unit creates an image of the food or drink whose exterior appearance is changed while an exterior appearance of the object is not changed.

15. The display control apparatus according to claim 1, wherein
the detection unit detects an image of a food plate in which the food or drink is served from the input image, and
the image creation unit creates an image of the food plate and the food or drink whose exterior appearances are changed.

16. The display control apparatus according to claim 1, wherein
the detection unit detects food or drink present in an eye gaze direction of a user, and
the image creation unit creates an image of the food or drink present in the eye gaze direction of the user detected by the detection unit, an exterior appearance of which is changed.

17. A recording medium having a program recorded thereon, the program causing a computer to function as:
a detection unit that detects whether or not an input image contains an image of food or drink;
an image creation unit that creates an image of food or drink whose exterior appearance is changed, when the detection unit detects an image of the food or drink; and
a display control unit that performs control to display the image created by the image creation unit on a display unit.
